# EUROPÄISCHE PATENTSCHRIFT

(11) **EP 0 889 721 B1**
(45) Veröffentlichungstag und Bekanntmachung des Hinweises auf die Patenterteilung: **17.04.2002**
(21) Anmeldenummer: 97919265.5
(22) Anmeldetag: 11.03.1997
(51) Int. Cl.: A61K 9/107, A61K 33/00, A61K 31/695

(54) **HEILMITTEL ZUR ÄUSSERLICHEN ANWENDUNG UND VERWENDUNG EINER WÄSSRIGEN ÖL-EMULSION FÜR EIN DERARTIGES HEILMITTEL**
REMEDY FOR EXTERNAL APPLICATION, AND USE OF AN OIL-IN-WATER EMULSION FOR SAID REMEDY
MEDICAMENT POUR APPLICATION EXTERNE ET UTILISATION D'UNE EMULSION HUILE DANS EAU POUR LEDIT MEDICAMENT

(30) Priorität: 25.03.1996 DE 19611742
(43) Veröffentlichungstag der Anmeldung: 13.01.1999
(73) Patentinhaber: Bauer, Wulf, 50968 Köln (DE)
(72) Erfinder: THÖNE, Gerd, D-32105 Bad Salzuflen (DE)
(74) Vertreter: Bauer, Wulf, Dr.
(86) Internationale Anmeldenummer: DE9700476
(87) Internationale Veröffentlichungsnummer: WO9735558

(56) Entgegenhaltungen:
- EP-A- 0 575 137
- FR-A- 2 726 187
- US-A- 4 917 891
- US-A- 5 169 429
- HARRY AUTERHOFF: "Lehrbuch der Pharmazeutischen Chemie" 1978 , WISSENSCHAFTLICHE VERLAGSGESELLSCHAFT MBH , STUTTGART XP002037313 *siehe Seite 39, 2ter Absatz*

## Beschreibung

Die vorliegende Erfindung betrifft ein Heilmittel für äußerliche Anwendung, insbesondere gegen Hauterkrankungen, und eine Verwendung einer Öl-Emulsion zur Herstellung eines derartigen Heilmittels.

Für eine Reihe von Hautkrankheiten wie z. B. Neuro Dermitis (Krätze), Psoriasis (Schuppenflechte), Akne, Kopfschuppen, Jucken, Herpes, Gürtelrose, Brandwunden, Wundschorf, Sonnenbrand und Venenschmerzen in den Beinen sind eine Reihe von Heilmitteln bekannt, nicht alle sind zufriedenstellend. Es besteht der Wunsch nach weiteren Heilmitteln für diesen Bereich.

Hautkrankheiten, wie beispielsweise die oben erwähnten Krankheiten, nehmen zu, dies mag an vielerlei Umwelteinflüssen und geänderten Umweltbedingungen liegen. Hauterkrankungen sind typischerweise mit sehr lästigen Begleiterscheinungen verbunden, es entsteht ein großer Juckreiz, der häufig unerträglich ist. Oft wird die Krankheit dadurch verschlimmert oder kommen andere Probleme hinzu, dass die befallenen Stellen zu stark gerieben oder aufgekratzt werden.

Hier setzt nun die Erfindung ein. Sie hat es sich zur Aufgabe gemacht, ein Heilmittel zur äußerlichen Anwendung, insbesondere gegen Hauterkrankungen, anzugeben, mit dem ein möglichst rasches Abheilen des Krankheitsbildes erreicht wird und das einfach in der Anwendung ist.

Gelöst wird diese Aufgabe ausgehend von dem Heilmittel der eingangs genannten Art durch eine Öl/Wasser-Emulsion, in der Mineralien in einer so kleinen Korngröße vorliegen, dass die Mineralien die Hautporen der menschlichen Haut passieren können, insbesondere amorphe pyrogene Kieselsäure in Öl gelöst, in Wasser emulgiert, mit Bestandteilen mineralischer Art aus Silicium, Calcium und/oder Magnesium.

Entscheidend sind die in der wäßrigen Ölemulsion enthaltenen, äußerst fein verteilten Mineralien. Sie sind so fein zerteilt, dass ihre Korngröße unterhalb des Porendurchmessers der Pore einer menschlichen Haut liegt. Dadurch können die feinstkörnigen Mineralien in die Haut eindringen. Die Mineralien sind also so fein, dass sie nicht oberflächlich liegen bleiben und somit abgefiltert werden, sondern in die Haut einziehen. Auf diese Weise werden diejenigen Mineralien in die Haut eingebracht, die der kranken Haut fehlen.

Als Öl hat sich insbesondere ein Mineralöl bewährt. In der Mineral-Öl-Emulsion befinden sich äußerst fein verteilt Mineralien wie Silicium, Calcium und in Spuren Magnesium. Die Emulsion ist nicht ionogen, pH-Wert ist 5 bis 7 neutral. Vorzugsweise liegt der Gehalt dieser Mineralien unter 1 Gew-%, vorzugsweise unter 0,1 Gew-% der Emulsion. Es können auch andere Mineralien vorhanden sein z.B. aus der Alkali- und/oder der Erdalkaligruppe. Die Mineralien sind nicht gelöst, sondern fein verteilt.

Als besonders bevorzugt hat sich die Zugabe eines Emulgators zur Grundmischung erwiesen, es werden handelsübliche Emulgatoren eingesetzt.

Weiterhin hat es sich als vorteilhaft erwiesen, der Emulsion ein Antiseptikum zuzufügen, hier eignet sich insbesondere Teebaumöl. Teebaumöl übernimmt in der Zusammensetzung neben der Rolle des Antiseptikums auch die Aufgabe der Hautberuhigung sowie der Beseitigung des Juckreizes.

Weiterhin hat es sich als vorteilhaft erwiesen, der Emulsion ein Tensid beizufügen, insbesondere ein anionisches Tensid 15 bis 30 %. Tenside reinigen die Verschmutzung der Haut und wirken zudem als Emulgator.

Als besonders vorteilhaft hat es sich herausgestellt, der Emulsion ein Glyzerin zuzufügen. Glyzerin ist bekannt als Feuchthaltemittel für die Haut, es wirkt zudem als eine Art Weichmacher für die Haut.

Um dem Heilmittel eine rückfettende Eigenschaft zu geben, wird vorzugsweise in Anwendungen, bei denen Rückfettung gewünscht wird, beispielsweise also nicht für Akne, ein rückfettendes Additiv hinzugesetzt. Hier kann auf handelsübliche Zusammensetzungen von Hautmilch für trockene Haut zurückgegriffen werden, insbesondere enthält der rückfettende Zusatz Öle, Parafine, Stearat usw.

Schließlich bezieht sich die Erfindung auf die Verwendung einer Öl-Wasser-Emulsion, der feinstkörnige Mineralien beigefügt sind, zur Herstellung eines Heilmittels für die äußere Anwendung, insbesondere gegen Hauterkrankungen. In einer bevorzugten Ausführung wird das Heilmittel so dünnflüssig zubereitet, dass es in Sprühflaschen versprüht werden kann.

Teebaumöl gibt es in technischen und pharmazeutischen Graden. Beide haben im erfindungsgemäßen Heilmittel gleiche Wirkungen. Die pharmazeutische Ausführung hat den Vorteil, nicht ganz so übel zu riechen.

200 Gramm Teebaumöl haben typisch folgende Bestandteile:

| | |
|---|---|
| terpinen-4-öl | 70 g |
| α-terpinöl | 14 g |
| τ-terpinen | 46 g |
| α- terpinen | 20 g |
| sigma-cymen | 20 g |
| 1,8 cineol | 20 g |
| α-pinen | 7 g |
| α-terpinolen | 7 g |
| d-limonen | 6 g |

Das Heilmittel hilft dem Körper zur Selbsthilfe. Das besprühte Gewebe wird warm, weil es stark durchblutet wird. Dadurch wird auch ein Teil der Heilung und Linderung bewirkt. Dies ist ein Vorteil, der eine Besonderheit des erfindungsgemäßen Heilmittels ist. Die stärkere Durchblutung erklärt die Wirkung des erfindungsgemäßen Heilmittels z.B. bei der Behandlung von Venenschmerzen.

### Beispiele

### Beispiel 1:

Ein Akne-Präparat hat die folgenden Bestandteile:
5.000 g Wasser entmineralisiert
500 g Minerallösung in Öl emulgiert
200 g Teebaumöl
10 g anionische Tenside 15 - 30 %
500 g Glyzerin

Bei Auftragen auf die Haut, insbesondere Aufsprühen, heilt Akne in zwei Stufen ab. Erst vertrocknen die bereits bestehenden Pickel, dann blüht die Haut aus, es sieht aus wie Masern mit zahlreichen roten Punkten. Sie entstehen, weil die verdeckten Fettpfropfen, die noch nicht entzündet sind, ausgetrieben werden. Danach ist die Haut glatt.

### Beispiel 2:

Für die Herstellung eines Mittels gegen Venenschmerzen in den Beinen:
5.000 g Wasser entmineralisiert
1.000 g Minerallösung in Öl emulgiert
100 g Teebaumöl
10 g anionische Tenside
1.000 g Glyzerin

Nach Venenoperationen fühlt man in den Beinen häufig brennende Schmerzen. Sie lassen nach dem Einsprühen mit dem Fluid nach. Eine Behandlung pro Tag reicht aus, um schmerzfrei zu bleiben.

### Beispiel 3:

Mittel gegen Neuro Dermitis:
5.000 g Wasser entmineralisiert
500 g Minerallösung in Öl emulgiert
200 g Teebaumöl
10 g anionische Tenside 15 - 30 %
500 g Glyzerin
500 g Hautmilch für trockene Haut aus Wasser, Octyl Stearat, Paraffinöl, Castor Öl, Sorbitan Oleat, Dimethicone Tocopherylacetet, Heliotropine, Phenoxyethanol, Magnesiumsulfat, Zinksulfat.

Der Juckreiz bei Neuro Dermitis, der unerträglich ist, verschwindet nach dem Einsprühen mit dem Mittel fast sofort, dadurch entfällt das Kratzen und Aufkratzen und die dadurch hervorgerufene, stetige Verschlimmerung des Krankheitsbildes. Die kranken Hautstellen verheilen sehr schnell, fast in Tagen. Die erkrankte, hornige Haut kann wie nach einem schweren Sonnenbrand abgezogen werden oder schuppt ab. Darunter kommt eine dünne, rote Haut zum Vorschein, die sehr schnell normal wird und nicht mehr befallen ist.

### Beispiel 4:

Für die Herstellung des erfindungsgemäßen Heilmittels haben Rezepturen mit den folgenden, angegebenen Bandbreiten sich als günstig erwiesen:
5.000 g Wasser
50-5.000 g Minerallösung in Öl emulgiert
10-2.000 g Teebaumöl
1-100 g anionische Tenside und
1-3.000 g Glyzerin.

Für das rückfettend wirkende Additiv der unter Beispiel 3 angegebenen Zusammensetzung hat sich eine Zugabe von 10 bis 2.000 g als günstig erwiesen.

### Beispiel 5:

| Novoskin® /Dermac® | Anteil |
|---|---|
| Wasser | 78 % |
| Glyzerin 85 % | 8 % |
| | |

| Silizium-Schlämme | |
|---|---|
| Silika | 2,45 % |
| Methylzellulose | 0,05 % |
| Wasser | 2,5 % |
| | |

| Kalzium-Schlämme | |
|---|---|
| Kalziumcarbonat | 0,74 % |
| Methylzellulose | 0,02 % |
| Wasser | 1,24 % |
| Castoröl | 2 % |
| Malaleucaöl | 2 % |
| Dexpanthenol | 1 % |
| | |

| Magnesium-Schlämme | |
|---|---|
| Magnesiumcarbonat | 0,2 % |
| Methylzellulose | 0,01 % |
| Wasser | 0,79 % |
| Avocadoöl | 1 % |

| Bestandteil | Anteil | Funktion | Standard |
|---|---|---|---|
| Wasser | 78 % | Lösungsmittel | normal |
| Glyzerin 85 % | 8 % | Weichmacher | Ph. Eur. |
| Gereinigtes Wasser | 4,53 % | Lösungsmittel | Ph. Eur. |
| Kolloidales Silikananhydrid(1) | 2,45 % | Scheuermittel | Ph. Eur. |
| Castoröl | 2 % | Steifmacher | U.S.N.F. |
| Malaleucaöl | 2 % | Duftstoff | normal |
| Dexpanthenol | 1 % | Provitamin | Ph. Eur. |
| Avocadoöl | 1 % | Duftstoff | normal |
| Kaliumcarbonat | 0,74 % | Scheuermittel | Ph. Eur. |
| Magnesiumcarbonat | 0,2 % | Scheuermittel | Ph. Eur. |
| Methylzellulose | 0,08 % | Suspensionsmittel | Ph. Eur. |

| | | | |
|---|---|---|---|
| (1) siehe oben: Silizium-Schlämme, sowie: Hollemann-Wiberg, *Lehrbuch der anorganischen Chemie,* 57.-70 Auflage, Walter der Gruyter & Co, Berlin 1964, S. 324 und 330. | | | |

### Beispiel 6:

| Novoskin® forte | |
|---|---|
| Wasser | 70 % |

| Silizium-Schlämme | |
|---|---|
| Silika | 4,9 % |
| Methylzellulose | 0,1 % |
| Wasser | 5 % |
| Glyzerin 85 % | 8 % |

| Kalzium-Schlämme | |
|---|---|
| Kalziumcarbonat | 1,48 % |
| Methylzellulose | 0,04 % |
| Wasser | 2,48 % |

| Magnesium-Schlämme | |
|---|---|
| Magnesiumcarbonat | 0,4 % |
| Methylzellulose | 0,02 % |
| Wasser | 1,58 % |
| Castoröl | 2 % |
| Malaleucaöl | 2 % |
| Dexpanthenol | 1 % |
| Avocadoöl | 1 % |

| Bestandteil | Anteil | Funktion | Standard |
|---|---|---|---|
| Wasser | 70 % | Lösungsmittel | normal |
| Gereinigtes Wasser | 9,06 % | Lösungsmittel | Ph. Eur. |
| Glyzerin 85 % | 8 % | Weichmacher | Ph. Eur. |
| Kollodiales Silikananhydrid | 4,9 % | Scheuermittel | Ph. Eur. |
| Castoröl | 2 % | Steifmacher | U.S.N.F. |
| Malaleucaöl | 2 % | Duftstoff | normal |
| Kaliumcarbonat | 1,48 % | Scheuermittel | Ph. Eur. |
| Dexpanthenol | 1 % | Provitamin | Ph. Eur. |
| Avocadoöl | 1 % | Duftstoff | normal |
| Magnesiumcarbonat | 0,4 % | Scheuermittel | Ph. Eur. |
| Methylzellulose | 0,16 % | Suspensionsmittel | Ph. Eur. |

### Beispiel 7:

| Novoskin® mild | |
|---|---|
| Wasser | 80 % |
| Glyzerin 85 % | 8 % |

| Silizium-Schlämme | |
|---|---|
| Silika | 2,45 % |
| Methylzellulose | 0,05 % |
| Wasser | 2,5 % |

| Kalzium-Schlämme | |
|---|---|
| Kalziumcarbonat | 0,74 % |
| Methylzellulose | 0,02 % |
| Wasser | 1,24 % |
| Castoröl | 2 % |
| Dexpanthenol | 1 % |

| Magnesium-Schlämme | |
|---|---|
| Magnesiumcarbonat | 0,2 % |
| Methylzellulose | 0,01 % |
| Wasser | 1,79 % |
| Avocadoöl | 1 % |

| Bestandteil | Anteil | Funktion | Standard |
|---|---|---|---|
| Wasser | 80 % | Lösungsmittel | normal |
| Glyzerin 85 % | 8 % | Weichmacher | Ph. Eur. |
| Gereinigtes Wasser | 4,53 % | Lösungsmittel | Ph. Eur. |
| Kollodiales Silikananhydrid | 2,45 % | Scheuermittel | Ph. Eur. |
| Castoröl | 2 % | Steifmacher | U.S.N.F. |
| Dexpanthenol | 1 % | Provitamin | Ph. Eur. |
| Avocadoöl | 1 % | Duftstoff | normal |
| Kaliumcarbonat | 0,74 % | Scheuermittel | Ph. Eur. |
| Magnesiumcarbonat | 0,2 % | Scheuermittel | Ph. Eur. |
| Methylzellulose | 0,08 % | Suspensionsmittel | Ph. Eur. |

Als Wasser kann verwendet werden destilliertes Wasser oder ein Wasser mit folgenden Inhaltsstoffen:

| | |
|---|---|
| Natrium | 5,4 mg/l or ppm |
| Kalium | 1,1 mg/l or ppm |
| Magnesium | 24,6 mg/l or ppm |
| Kalzium | 49,8 mg/l or ppm |
| Chloride | 11,4 mg/l or ppm |
| Sulphate | 7,1 mg/l or ppm |
| Hydrogencarbonate | 253,0 mg/l or ppm |

### Beispiel 8:

### Zusammensetzung der Öl-Emulsion mit Mineralien

38 % Festkörper, End-Korngröße der Mineralien im Bereich 1 bis 100 Manometer, vorzugsweise 1 bis 10 Nanometer.
10 - 30 = 20 % Quarzmehl, Anfangsgröße 5 Mikrometer
5 - 30 = 14 % Dolomitsteinmehl, Anfangsgröße 2 Mikrometer
1 - 10 = 3 % Magnesiumpulver ca 3 Mikrometer
01 - 5 = 1 % Methylcellulose
10 - 60 = 38 % Rizinusöl, Pharmagrad
2- 40 = 22 % Wasser

Diese Mischung wird zunächst in einem Kneter intensiv mit einer Temperatur von ca. 50° C geknetet und dann - inline - einer Kolloidmühle zugeführt. Die Kolloidmühle hat eine Kreuzverzahnung mit Umpumpvorrichtung und Rotorkühlung. Im langsamen Durchgang werden die Mineralien bis auf die Größe von 10⁻⁹m zermahlen. Das Rizinusöl emulgiert im warmen Wasser und wird durch die Methylcellulose stabilisiert. Um Überhitzung zu vermeiden ist im Mahlstadium eine Kühlung des Mahlkopfes notwendig. Die so gewonnene Mineral-Öl-Emulsion ist stabil und läßt sich mit Wasser weiter verdünnen und einarbeiten.

## Patentansprüche

1. Heilmittel zur äußerlichen Anwendung, insbesondere gegen Hauterkrankungen, **gekennzeichnet durch** eine Öl/Wasser-Emulsion, insbesondere Mineralöl/Wasser-Emulsion, in der Mineralien in einer so kleinen Korngröße, vorzugsweise kleiner 100 Nanometer, vorliegen, dass die Mineralien die Hautporen der menschlichen Haut passieren können, insbesondere amorphe pyrogene Kieselsäure in Öl gelöst, in Wasser emulgiert, mit Bestandteilen mineralischer Art aus Silicium, Calcium und/oder Magnesium.

2. Heilmittel nach Anspruch 1, **dadurch gekennzeichnet, dass** der Emulsion ein Emulsionsstabilisator, vorzugsweise Carboxymethylcellulose, insbesondere in einem Bereich von 0,025 bis 0,75 Gew-% bezogen auf die Gesamtemulsion, beigemischt ist.

3. Heilmittel nach Anspruch 1, **dadurch gekennzeichnet, dass** der Emulsion ein Antiseptikum, beispielsweise Teebaumöl, zugefügt ist, insbesondere dass das Antiseptikum 0,001 bis 1 Gew-% bezogen auf die Emulsion einnimmt.

4. Heilmittel nach Anspruch 1, **dadurch gekennzeichnet, dass** ein Tensid, beispielsweise ein anionisches Tensid 15 bis 30 % beigefügt ist, vorzugsweise in einer Menge von 0,0001 bis 1 Gew-% bezogen auf das Gewicht der Emulsion.

5. Heilmittel nach Anspruch 1, **dadurch gekennzeichnet, dass** ein Glyzerin beigefügt ist, insbesondere in einer Menge von 0,001 bis 50 Gew-%, bezogen auf das Gewicht der Emulsion.

6. Heilmittel nach Anspruch 1, **dadurch gekennzeichnet, dass** eine ölhaltige, rückfettende Hautmilch zugesetzt ist, insbesondere in einem Gewichtsbereich von 0,001 bis 30 % bezogen auf das Gewicht der Emulsion.

7. Heilmittel nach Anspruch 1, **dadurch gekennzeichnet, dass** die Mineralien mind. 1 Mineral aus der folgenden Gruppe umfassen: Silicium, Calcium, Magnesium.

8. Heilmittel nach Anspruch 1, **dadurch gekennzeichnet, dass** es für einen Sprühauftrag zubereitet ist, insbesondere 0,2 - 1 % Minerallösung in Öl, Rest Wasser, in der Emulsion aufweist.

9. Verwendung einer wäßrigen Emulsion aus pyrogenen Mineralien, eines Emulgators und Mineralien, die in einer so kleinen Korngröße vorliegen, dass sie Hautporen der menschlichen Haut passieren können, zur Herstellung eines Heilmittels zur äußerlichen Anwendung, insbesondere gegen Hauterkrankungen.

10. Verwendung gemäß Anspruch 9, **dadurch gekennzeichnet, dass** als Verteilorgan eine Sprühflasche eingesetzt wird.

## Claims

1. Remedy for external application, in particular for the treatment of skin diseases, **characterized by** an oil-in-water emulsion, in particular a mineral oil-in-water emulsion, comprising minerals the grain size thereof being so small, especially smaller than 100nm, that the minerals can pass through the skin pores of human skin, in particular amorphous pyrogenic silicic acid dissolved in oil and emulsified in water with mineral ingredients such as silicon, calcium and/or magnesium.

2. Remedy according to claim 1, **characterized in that** a stabilizer is used in the composition of the emulsion, preferably carboxymethylcellulose, in particular in a range of 0,025 to 0,75% by weight of the total composition.

3. Remedy according to claim 1, **characterized in that** an antiseptic, for example tea tree oil is added to the emulsion, in particular that the antiseptic is present in an amount between 0,001 to 1% by weight of the total composition.

4. Remedy according to claim 1, **characterized in that** a surface-active agent, for example an anionic surface-active agent 15 to 30% is added, preferably in an amount between 0,0001 to 1% by weight of the weight of the total composition.

5. Remedy according to claim 1, **characterized in that** glycerin is added, in particular in an amount between 0,001 to 50% by weight of the weight of the total composition.

6. Remedy according to claim 1, **characterized in that** an oil emulsive, fat-restoring skin milk is added to the composition, in particular in a weight range of 0,001 to 30% of the weight of the total composition.

7. Remedy according to claim 1, **characterized in that** the minerals include at least one mineral selected from the group consisting of silicon, calcium, magnesium.

8. Remedy according to claim 1, **characterized in that** the remedy is prepared for being atomized, the emulsion containing in particular 0,2 - 1% mineral solution in oil, the rest water.

9. Use of an aqueous emulsion consisting of pyrogenic minerals, an emulsifier and minerals, the grain size thereof being so small that the minerals can pass through the skin pores of human skin, for the production of a remedy for external application, in particular for the treatment of skin diseases.

10. Use according to claim 9, **characterized in that** an atomizer is used to apply the remedy.

## Revendications

1. Remède pour l'usage externe, en particulier contre des maladies de la peau, **caractérisé par** une émulsion d'huile dans l'eau, en particulier par une émulsion d'huile minérale dans l'eau, dans laquelle des minéraux sont présents en une grosseur de grain tellement petite, de préférence inférieure à 100 nanomètres, que les minéraux peuvent passer les pores de la peau humaine, en particulier par un acide silicique amorphe pyrogène dissous dans l'huile, émulsionné dans l'eau, avec des composants de type minéral en silicium, en calcium et/ou en magnésium.

2. Remède selon la revendication 1, **caractérisé par le fait que** l'on ajoute à l'émulsion un stabilisant d'émulsion, de préférence de la carboxyméthylcellulose, en particulier dans une gamme comprise entre 0,025 et 0,75 % en poids par rapport à l'émulsion totale.

3. Remède selon la revendication 1, **caractérisé par le fait que** l'on ajoute à l'émulsion un antiseptique, par exemple de l'huile de l'arbre à thé, en particulier que l'antiseptique occupe entre 0,001 et 1 % en poids par rapport à l'émulsion.

4. Remède selon la revendication 1, **caractérisé par le fait que** l'on ajoute un agent de surface, par exemple un agent de surface anionique 15 à 30 %, de préférence en une quantité comprise entre 0,0001 et 1 % en poids par rapport au poids de l'émulsion.

5. Remède selon la revendication 1, **caractérisé par le fait que** l'on ajoute une glycérine, en particulier en une quantité comprise entre 0,001 et 50 % en poids par rapport au poids de l'émulsion.

6. Remède selon la revendication 1, **caractérisé par le fait que** l'on ajoute un lait de beauté huileux rendant de la graisse, en particulier dans une gamme de poids comprise entre 0,001 et 30 % par rapport au poids de l'émulsion.

7. Remède selon la revendication 1, **caractérisé par le fait que** les minéraux comprennent au moins un minéral choisi dans le groupe suivant comprenant le silicium, le calcium, le magnésium.

8. Remède selon la revendication 1, **caractérisé par le fait qu'**il est préparé pour être appliqué par pulvérisation, qu'il présente en particulier entre 0,2 et 1 % de solution minérale dans l'huile, reste eau, dans l'émulsion.

9. Utilisation d'une émulsion aqueuse en minéraux pyrogènes, d'un émulsifiant et de minéraux qui sont présents en une grosseur de grain tellement petite qu'ils peuvent passer les pores de la peau humaine, pour la production d'un remède pour l'usage externe, en particulier contre des maladies de la peau.

10. Utilisation selon la revendication 9, **caractérisée par le fait qu'**un atomiseur est utilisé pour appliquer le remède.
